# EUROPEAN PATENT SPECIFICATION

(11) **EP 4 115 907 B1**
(45) Date of publication and mention of the grant of the patent: **06.05.2026**
(21) Application number: 21761537.6
(22) Date of filing: 01.03.2021
(51) Int. Cl.: A61K 31/519, G01N 33/15, C07D 487/04, C12N 9/99, C07D 471/04, A61P 43/00, A61P 35/00, A61P 1/18, A61K 31/506, G01N 33/50, A61K 31/4409, A01K 67/68, A61K 45/06

(54) **COMBINATION DRUG OF KINASE INHIBITORS FOR USE IN THE TREATMENT OF PANCREATIC CANCER**
KOMBINATIONSARZNEIMITTEL VON KINASEINHIBITOREN ZUR BEHANDLUNG VON PANKREASKREBS
MÉDICAMENT COMBINÉ D'INHIBITEURS DE KINASES POUR LE TRAITEMENT DU CANCER DU PANCRÉAS

(30) Priority: 27.02.2020 JP 2020032112
(43) Date of publication of application: 11.01.2023
(73) Proprietor: National University Corporation Hokkaido University, Sapporo-shi, Hokkaido 060-0808 (JP)
(72) Inventor: SONOSHITA Masahiro, Sapporo-shi, Hokkaido 060-0808 (JP); SEKIYA Sho, Sapporo-shi, Hokkaido 060-0808 (JP); HIRANO Satoshi, Sapporo-shi, Hokkaido 060-0808 (JP)
(74) Representative: Wasner, Marita
(86) International application number: PCT/JP2021/007651
(87) International publication number: WO 2021/172582

(56) References cited:
- WO-A1-2012/167247
- WO-A1-2015/041533
- WO-A1-2018/183891
- WO-A1-2018/203219
- WO-A1-2020/010280
- WO-A2-2009/074827
- JP-A- 2012 500 013
- JP-A- 2014 525 464
- JP-A- 2016 532 452
- JP-A- 2016 539 156
- JP-A- 2019 206 516
- US-A1- 2019 011 435
- WALTERS DUSTIN M ET AL: "Inhibition of the Growth of Patient-Derived Pancreatic Cancer Xenografts with the MEK Inhibitor Trametinib Is Augmented by Combined Treatment with the Epidermal Growth Factor Receptor/HER2 Inhibitor Lapatinib", NEOPLASIA, NEOPLASIA PRESS, ANN ARBOR, MI, US, vol. 15, no. 2, 1 February 2013 (2013-02-01), pages 143 - 155, XP008165494, ISSN: 1522-8002, DOI: 10.1593/NEO.121712
- HUA ZHONG ET AL: "Synergistic Effects of Concurrent Blockade of PI3K and MEK Pathways in Pancreatic Cancer Preclinical Models", PLOS ONE, vol. 8, no. 10, 9 October 2013 (2013-10-09), pages e77243, XP055313720, DOI: 10.1371/journal.pone.0077243

## Description

### FIELD

The present invention pertains to a pharmaceutical combination of at least two types of kinase inhibitors for treatment of pancreatic cancer, and a kinase inhibitor for use in the pharmaceutical combination.

### BACKGROUND

Pancreatic cancer is one of the most difficult cancers to treat. At present, pancreatic cancer is the fourth leading cause of cancer death and expected to be second within 10 years, raising concerns about major social problems.

Pancreatic ductal carcinoma developing in the pancreatic duct accounts for 90% of pancreatic cancer or more, and is characterized by the absence of subjective symptoms even after onset. For this reason, patients frequently miss the opportunity for diagnosis and have cancer cells metastasized to other organs when diagnosed. Metastasis accounts for poor prognosis. Therefore, the survival rate of pancreatic cancer patients is the lowest among the patients of all types of cancer. Therefore, development of therapies for prevention and treatment of pancreatic cancer has been an extremely important issue for many years.

Pancreatic cancer is known to show extremely high resistance to drug therapy, and development of new drugs has been extremely difficult. Even the few approved drugs such as gemcitabine, an antimetabolic agent, and erlotinib, an EGFR inhibitor, have been pointed out to have problems such as insufficient efficacy and toxicity.

Pancreatic cancer frequently harbors four gene mutations, which are KRAS gene mutation inducing KRAS activation, TP53 gene mutation inducing TP53 inactivation, CDKN2A gene mutation inducing CDKN2A inactivation and SMAD4 gene mutation inducing SMAD4 inactivation. Patients with pancreatic cancer with the poorest prognosis are known to have all of the above four gene mutations (Non-Patent Literature 1). Therefore, animal models with the above four gene mutations and research on therapies by using the animal models are of great importance in developing therapies for pancreatic cancer. However, to date, no animal model mimicking the abnormality of these four genes has been produced, and therefore remaining major obstacle in the research field.

In recent years, use of Drosophila as an animal model has been attracting attentions. As a useful animal model Drosophila has the following characteristics: high genetic conservation with mammals (for example, 75% or more of genes altered in human diseases are present also in Drosophila); conserved internal structures (epithelial structure, main organs and the like) functionally corresponding to those in mammals; a wide variety of genetic analysis tools (siRNA knockdown lines and mutants for almost all genes are available); and rapid and inexpensive rearing (in 10 days for production of next generation, rearing cost at one-thousandth of that for mice).

Inventors of the present invention reported on the exploration for kinases involved in medullary thyroid cancer and screening of anticancer agents basing upon the kinase information with use of a Drosophila strain ptc>dRet^{M955T}, which expresses a mutant form of RET, a receptor tyrosine kinase associated with medullary thyroid cancer, as an animal model (Patent Literature 1, Non-Patent Literatures 2 to 4). The ptc>dRet^{M955T} fly is modified to express the RET mutant in epithelial cells localized to a wing disc of larva with use of ptc promotor and gal4-UAS system, which is a binary system capable of forcing the expression of foreign genes in Drosophila. ptc>dRet^{M955T} flies exhibit the property of producing tumor-like lesions that cause all individuals to die without reaching adulthood.

Thus, using Drosophila as an animal model is an effective means for developing cancer therapies. However, since the ptc>dRet^{M955T} fly is a model for medullary thyroid cancer, it is necessary to create a new animal model for pancreatic cancer in order to explore therapies for pancreatic cancer.

### CITATION LIST

### PATENT LITERATURE

Patent Literature 1: Japanese Translation of PCT Application Publication No. 2019-528279

### NON PATENT LITERATURES

Non Patent Literature 1: Qian et al., JAMA Oncol. 2018; 4(3): e173420.
Non Patent Literature 2: Sonoshita et al., Curr. Top. Dev. Biol. 2017; 121:287-309
Non Patent Literature 3: Sonoshita et al., Nat. Chem. Biol. 2018; 14(3): 291-298.
Non Patent Literature 4: Ung, Sonoshita et al., PLoS Comput. Biol. 2019; 15(4): e1006878.

WO2018203219 relates to a pharmaceutical combination comprising (a) a Raf inhibitor and (b) a MEK inhibitor. WO2012167247 relates to methods for treatment of various cell proliferative disorders by administering a MEK inhibitor in combination with a selective inhibitor of Aurora A kinase. Walters et al relates to inhibition of growth of patient derived pancreatic cancer cell lines with Trametinib (MEK inhibitor) combined with lapatinib (EGFR/HER2 inhibitor). WO2020010280 relates to methods for treating pancreatic cancer using a MEK inhibitor and a CDK4/6 inhibitor. WO2015041533 relates to combined use of inhibitors of ROCK protein and proteins of the MAPK/ERK-pathway in the treatment of certain types of cancer.

### SUMMARY

### TECHNICAL PROBLEM

Among the four genes in which the above mutations have been identified in human pancreatic cancer, the CDKN2A gene does not exist in Drosophila. Therefore, a Drosophila model for pancreatic cancer cannot be created by directly reproducing the gene mutations identified in human pancreatic cancer in Drosophila, unlike a Drosophila model for medullary thyroid cancer. The present disclosure is aimed at creating Drosophila that can serve as a model for pancreatic cancer, as well as providing a method for searching a novel therapeutic means for pancreatic cancer using Drosophila and a medicine for use in treatment of pancreatic cancer.

### SOLUTION TO PROBLEM

Inventors of the present invention have created a Drosophila strain with characteristics corresponding to the four gene mutations associated with human pancreatic cancer, and through screening using the strain, found that a combination of plural types of kinase inhibitors is effective in the treatment of pancreatic cancer, and disclosed the following information as references.
(1) A method for screening an anticancer agent, the method comprising:
   a step of making a test substance be ingested by Drosophila having the following characteristics a) to d):
      a) expression of a mutant Ras85D in which glycine is substituted with aspartic acid, valine or cysteine at 12th position of the amino acid sequence of SEQ ID NO: 1,
      b) deletion or suppressed expression of a p53 gene,
      c) overexpression of a Cyclin E gene, and
      d) deletion or suppressed expression of a Med gene;
   a step of measuring survival rate of Drosophila ingesting the test substance; and
   a step of selecting the test substance as a candidate substance for the anticancer agent when the survival rate of Drosophila ingesting the test substance is higher than that of Drosophila not ingesting the test substance.
(2) The method according to (1), wherein the mutant Ras85D is a protein in which glycine is substituted with aspartic acid at 12th position of the amino acid sequence of SEQ ID NO: 1, the p53 gene expression is suppressed by introducing a nucleic acid suppressing the p53 gene expression, the Cyclin E is overexpressed by introducing a nucleic acid encoding Cyclin E, and the Med gene expression is suppressed by introducing a nucleic acid suppressing the Med gene expression.
(3) The method according to (1), wherein the Drosophila is a Drosophila into which a nucleic acid encoding the mutant Ras85D in which glycine is substituted with aspartic acid at 12th position of the amino acid sequence of SEQ ID NO: 1, a p53 gene knockdown nucleic acid, a nucleic acid encoding a Cyclin E gene and a Med gene knockdown nucleic acid are introduced.
(4) A method for screening an anticancer agent, the method comprising:
   a step of rearing, on a food containing a test substance, an egg resulting from mating Drosophila into which a gal4 gene is introduced to Drosophila into which the following nucleic acids a') to d') are introduced:
      a') a nucleic acid having a nucleotide sequence encoding a mutant Ras85D in which glycine is substituted with aspartic acid at 12th position of the amino acid sequence of SEQ ID NO: 1 in a downstream of a UAS sequence,
      b') a nucleic acid having a nucleotide sequence encoding shRNA for a p53 gene in the downstream of a UAS sequence,
      c') a nucleic acid having a nucleotide sequence encoding a Cyclin E gene in the downstream of a UAS sequence, and
      d') a nucleic acid having a nucleotide sequence encoding shRNA for a Med gene in the downstream of a UAS sequence;
   a step of measuring survival rate of Drosophila reared on the food containing the test substance; and
   a step of selecting the test substance as a candidate substance for the anticancer agent when the survival rate of Drosophila reared on the food containing the test substance is higher than that of Drosophila reared on a food not containing the test substance.
(5) The method according to any one of (1) to (4), wherein a rearing temperature for Drosophila is adjusted to control the survival rate of Drosophila not ingesting the test substance or Drosophila reared on a food not containing the test substance.
(6) A Drosophila strain having the following characteristics a) to d):
   a) expression of a mutant Ras85D in which glycine is substituted with aspartic acid, valine or cysteine at 12th position of the amino acid sequence of SEQ ID NO: 1,
   b) deletion or suppressed expression of a p53 gene,
   c) overexpression of a Cyclin E gene, and
   d) deletion or suppressed expression of a Med gene.
(7) The Drosophila strain according to (6), wherein the strain is introduced with the following nucleic acids a') to d'):
   a') a nucleic acid having a nucleotide sequence encoding a mutant Ras85D in which glycine is substituted with aspartic acid at 12th position of the amino acid sequence of SEQ ID NO: 1 in a downstream of a UAS sequence,
   b') a nucleic acid having a nucleotide sequence encoding shRNA for a p53 gene in the downstream of a UAS sequence,
   c') a nucleic acid having a nucleotide sequence encoding a Cyclin E gene in the downstream of a UAS sequence, and
   d') a nucleic acid having a nucleotide sequence encoding shRNA for a Med gene in the downstream of a UAS sequence.
(8) A pharmaceutical combination of at least two types of kinase inhibitors selected from the group consisting of a MEK inhibitor, an FRK inhibitor, a WEE inhibitor, an AURK inhibitor and a ROCK inhibitor for treatment of pancreatic cancer.
(9) A pharmaceutical combination of a MEK inhibitor and at least one type of kinase inhibitor selected from the group consisting of an FRK inhibitor, a WEE inhibitor, an AURK inhibitor and a ROCK inhibitor for treatment of pancreatic cancer.
(10) The pharmaceutical combination according to (8) or (9), wherein the MEK inhibitor is Trametinib.
(11) The pharmaceutical combination according to any one of claims (8) to (10), wherein the FRK inhibitor is AD80, the WEE inhibitor is Adavosertib, and the AURK inhibitor is Alisertib or BI-831266.
(12) A kinase inhibitor for use in combination with a MEK inhibitor for treatment of pancreatic cancer, the kinase inhibitor being selected from the group consisting of an FRK inhibitor, a WEE inhibitor, an AURK inhibitor and a ROCK inhibitor.
(13) The kinase inhibitor according to (12), wherein the MEK inhibitor is Trametinib.
(14) The kinase inhibitor according to claim 12 or 13, wherein the FRK inhibitor is AD80, the WEE inhibitor is Adavosertib, and the AURK inhibitor is Alisertib or BI-831266.
(15) A MEK inhibitor for use in combination with at least one type of kinase inhibitor selected from the group consisting of an FRK inhibitor, a WEE inhibitor, an AURK inhibitor and a ROCK inhibitor, for treatment of pancreatic cancer.
(16) The MEK inhibitor according to (15), wherein the MEK inhibitor is Trametinib.
(17) The MEK inhibitor according to (15) or (16), wherein the FRK inhibitor is AD80, the WEE inhibitor is Adavosertib, and the AURK inhibitor is Alisertib or BI-831266.

### EFFECT OF INVENTION

According to the present disclosure, Drosophila having characteristics corresponding to the four gene mutations associated with human pancreatic cancer can be produced. With use of this Drosophila as an animal model for human pancreatic cancer, substances capable of exhibiting a therapeutic effect on pancreatic cancer can be searched efficiently at a reduced cost. As well, combinations of two or more types of specific kinase inhibitors can be provided as a therapeutic agent for pancreatic cancer. The invention is set out in the appended set of claims.

### BRIEF DESCRIPTION OF DRAWINGS

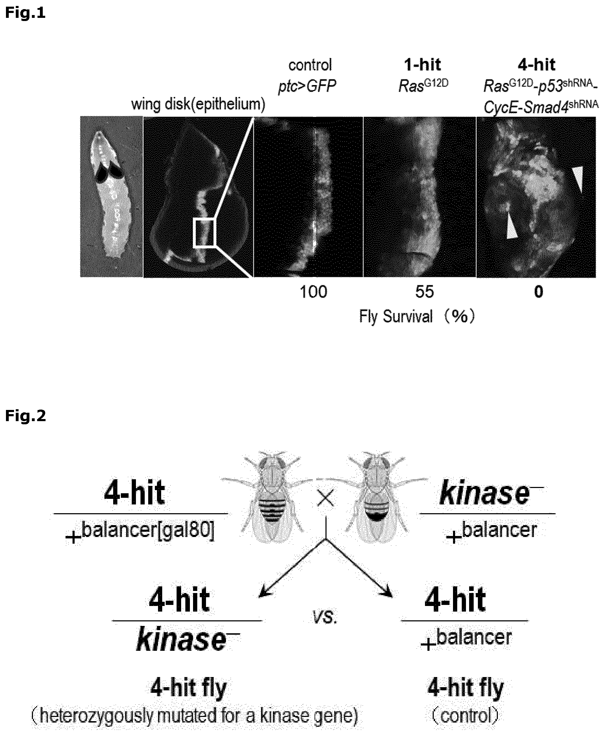

Fig. 1 illustrates fluorescent microscope images obtained by observing wing discs of larvae of control Drosophila (ptc>GFP), Drosophila expressing mutant Ras85D frequently found in pancreatic cancer cells (1-hit fly), and Drosophila mimicking four gene mutations frequently found in pancreatic cancer cells (4-hit fly).
Fig. 2 is a schematic diagram illustrating the protocol to produce 4-hit flies with heterozygous kinase mutation by introducing a heterozygous kinase gene mutation into a 4-hit fly.

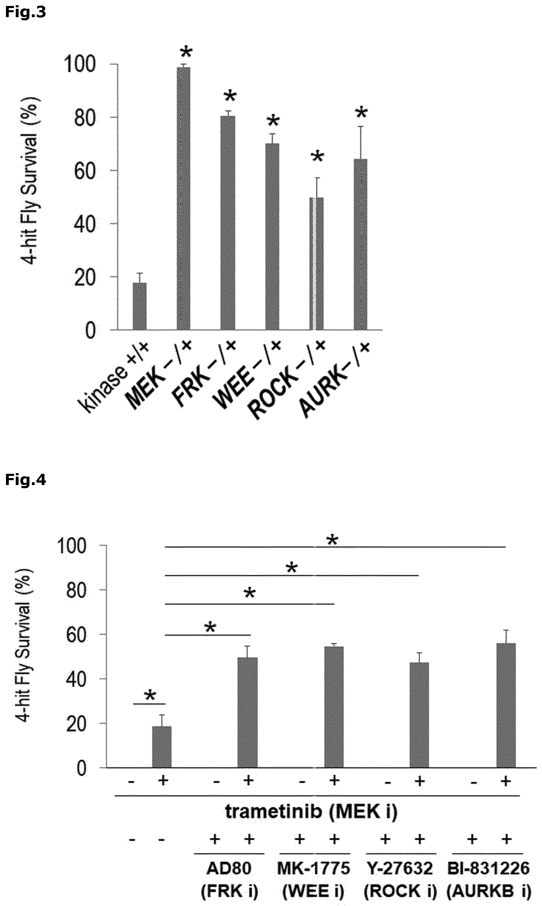

Fig. 3 is a graph illustrating survival rates of 4-hit flies with a heterozygous MEK, FRK, WEE, ROCK, or AURK mutation.
Fig. 4 is a graph illustrating survival rates of 4-hit flies (non-GFP) ingesting one of MEK inhibitor (Trametinib), FRK inhibitor (AD80), WEE inhibitor (MK-1775), ROCK inhibitor (Y-27632) and AURK inhibitor (BI-831266) alone and those ingesting a combination of MEK inhibitor with any one of other four kinase inhibitors. *p < 0.01.

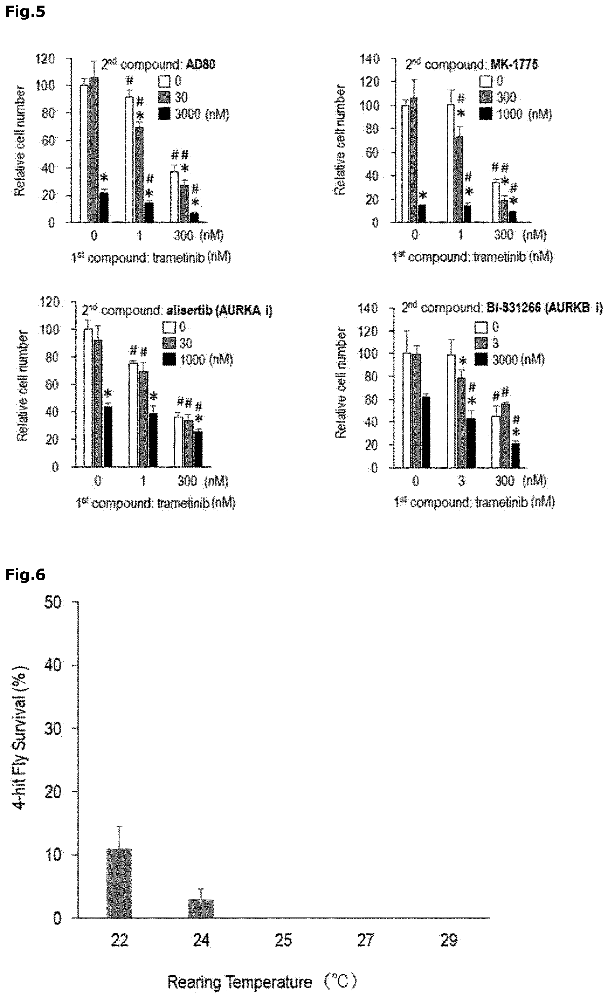

Fig. 5 is a set of graphs illustrating relative cell numbers of MIA PaCa-2 human pancreatic cancer cells cultured in the presence of combinations of MEK inhibitor (Trametinib) as 1st compound and one of FRK inhibitor (AD80), WEE inhibitor (MK-1775) and AURK inhibitor (Alisertib, BI-831266) as 2nd compound. * indicates a significant decrease compared to those without 2nd compound (0 nM) (p < 0.05). # indicates a significant decrease compared to those without Trametinib (0 nM) (p < 0.05).
Fig. 6 is a graph illustrating survival rates of 4-hit flies (non-GFP) reared at 22 to 29°C.

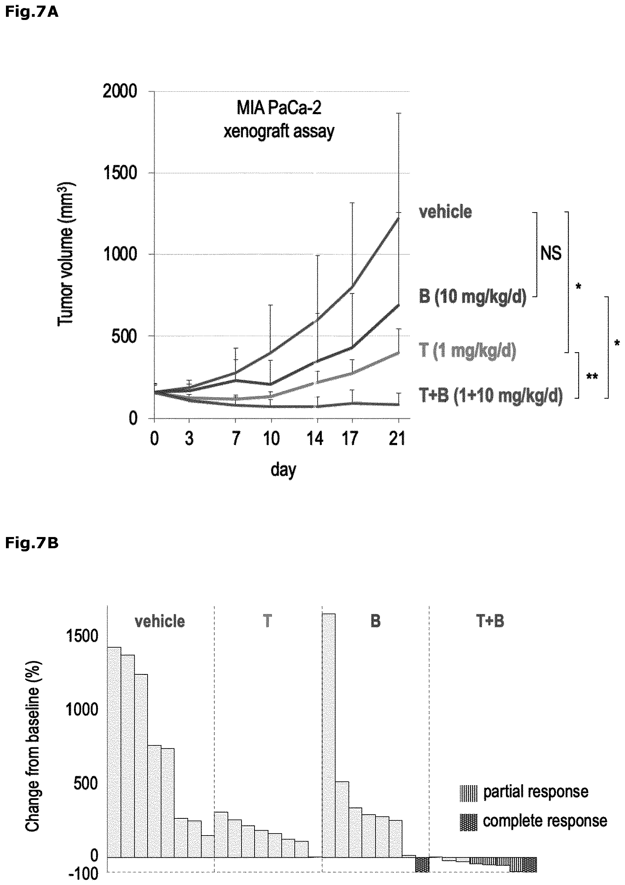

Fig. 7A is a graph illustrating changes in tumor volumes over time in tumor-bearing mice to which MEK inhibitor (Trametinib) alone, AURK inhibitor (BI-831266) alone or a combination thereof were orally administered. *p < 0.05, **p < 0.01 (Mann-Whitney U test conducted on the 21st day after administration). NS stands for not significant (no significant difference). Error bars indicate standard deviation of eight individuals.
Fig. 7B is a waterwall plot illustrating percentage changes compared between tumor volumes at the time of administration and that on the 21st day after administration for tumor-bearing mice to which MEK inhibitor (Trametinib) alone, AURK inhibitor (BI-831266) alone and a combination thereof were orally administered. One bar represents the percentage change in tumor volume of one mouse.

In the Figs above, the parts not falling within the scope of the claims are disclosed as references.

### DESCRIPTION OF EMBODIMENTS

### Drosophila mimicking human pancreatic cancer

First aspect of the present disclosure pertains to Drosophila with the following characteristics a) to d):
a) expression of a mutant Ras85D in which glycine is substituted with aspartic acid, valine or cysteine at 12th position in the amino acid sequence of SEQ ID NO: 1,
b) deletion or suppressed expression of a p53 gene,
c) overexpression of a Cyclin E gene, and
d) deletion or suppressed expression of a Med gene

Ras85D protein is a Drosophila orthologue of human KRAS. An amino acid sequence (SEQ ID NO: 1) of Ras85D and a nucleotide sequence encoding Ras85D are registered with NCBI under the accession numbers NP_476699.1 and NM_057351.5, respectively.

Mutations in codon 12 of human KRAS gene exon 2 are known as cancer-inducible mutations that lead to KRAS activation, and are frequently observed in pancreatic cancer cells. The mutant Ras85D is obtained by introducing a mutation corresponding to the cancer-inducible mutation of human KRAS into Ras85D, specifically by substituting glycine with aspartic acid, valine or cysteine at 12th position in the amino acid sequence represented in SEQ ID NO: 1, and functions as an activated KRAS. Forced expression of the gene encoding the mutant Ras85D in Drosophila is considered to enable mimicking a similar condition in Drosophila to that caused by an activated KRAS in human.

Drosophila p53 is an ortholog of human TP53. A nucleotide sequence encoding Drosophila p53 is registered with NCBI under the accession number NM_206545.2. In humans, it is known that p53 dysfunction resulting from TP53 gene mutation promotes generation and progression of cancers. The p53 mutation is frequently observed also in pancreatic cancer cells. Suppression of functional p53 gene expression in Drosophila, for example, deletion or suppressed expression of p53 gene is considered to enable mimicking a similar condition in Drosophila to that caused by inactivated p53 in human.

Cyclin-dependent kinase inhibitor 2A (CDKN2A), which is referred also to as P16, is a protein involved in regulation of cell cycle. It is known that inactivation of CDKN2A resulting from CDKN2A gene mutation is associated with generation of various cancers and frequently observed in pancreatic cancer cells. Because of no Drosophila orthologue of human CDKN2A, overexpression of Drosophila Cyclin E is used in the present disclosure instead of CDKN2A gene mutation. Cyclin E is a regulatory factor for progression of the G1 phase and transition to the S phase in the cell cycle, and is known to bind to cyclin dependent kinase 2 (CDK2) for its activation. The overexpression of Cyclin E in Drosophila functionally substitutes for CDKN2A inactivation, and according to a report (Datar et al. EMBO J. 19:4543, 2000), enables mimicking a similar condition in Drosophila to that caused by the CDKN2A inactivation. A nucleotide sequence encoding Drosophila Cyclin E is registered with NCBI under the accession number NP_476959.1.

Med (Mothers against decapentaplegic) is a Drosophila ortholog of human SMAD4. A nucleotide sequence encoding Med is registered with NCBI under the accession number NM_079871.4. In humans, SMAD4 is involved in TGF-β signaling, which is an inhibitory regulator of cell proliferation. Inactivation of SMAD4 resulting from SMAD4 gene mutation is noted as being involved with generation of cancers, and is frequently observed in pancreatic cancer cells. Suppression of functional Med gene expression in Drosophila, for example, deletion or suppressed expression of Med gene is considered to enable mimicking a similar condition in Drosophila to that caused by inactivated SMAD4 in human.

In the present disclosure, introduction of a mutation into a gene, deletion of a gene, and suppression of gene expression can be performed by using molecular biological methods generally used in the art.

In a preferred embodiment, the Drosophila has a mutant Ras85D in which glycine is substituted with aspartic acid at 12th position (represented as Ras^{G12D}), p53 gene expression in the Drosophila is suppressed by introducing a nucleic acid suppressing p53 gene expression, Cyclin E is overexpressed by introducing a nucleic acid encoding Cyclin E, and Med gene expression in the Drosophila is suppressed by introducing a nucleic acid suppressing Med gene expression.

Examples of the nucleic acid suppressing the gene expression used in the present disclosure include knockdown nucleic acids (antisense nucleic acid, siRNA, its precursor shRNA, shDNA encoding shRNA and the like) such as an antisense nucleic acid and siRNA. The knockdown nucleic acid can be designed as appropriate with reference to a nucleotide sequence of a target gene so as to be specific to the target gene and achieve a lowered off-target effect. In a further preferred embodiment, the Drosophila is a Drosophila into which a nucleic acid encoding Ras^{G12D}, a p53 gene knockdown nucleic acid, a nucleic acid encoding Cyclin E gene, and a Med gene knockdown nucleic acid are introduced. The four nucleic acids may be configured so that they all exist under one regulatory sequence, or each may exist under a different regulatory sequence from each other.

The four nucleic acids are preferably incorporated into a Drosophila genome along with a regulatory system that allows the timing and extent of their expression to be controlled. The Gal4-UAS system is typically used to achieve this preference. The Gal4-UAS system is a binary system in which a yeast transcription activation factor gal4 and its target sequence UAS (upstream activating sequence) are combined to induce forced gene expression. For example, a Drosophila in which a construct with the four nucleic acids placed downstream of the UAS is incorporated into the genome is mated with a Drosophila expressing gal4 protein (gal4 driver line) to obtain F1 Drosophila. The expression of the four nucleic acids in the F1 Drosophila can be controlled according to an expression pattern of the gal4 protein. In the gal4-UAS system, transcription capability of the gal4 protein varies depending on temperature. Therefore, by adjusting a rearing temperature for Drosophila, the activity of gal4 protein can be controlled, and the expression levels of the four nucleic acids can be controlled. The extent of expression of the four nucleic acids in the F1 Drosophila may be different depending on the cell and tissue having a promotor/enhancer region with an activity to control the gal4 expression, as well as on the level of the activity of the promotor/enhancer region in the cell and tissue. Accordingly, the expression levels of the four nucleic acids in the F1 can also be controlled by appropriately setting the promotor/enhancer region that controls the gal4 expression in the gal4 driver line, which is a parent to produce the F1. All of the above controls can be carried out within the scope of implementation capability for a person skilled in the art.

Positions into which the four nucleic acids are incorporated in the Drosophila genome are not particularly limited, and are preferably incorporated into chromosomes 2 or 3, which are autosomes.

In a further preferred embodiment, the Drosophila is a Drosophila into which the following nucleic acids a') to d') are introduced:
a') a nucleic acid having a nucleotide sequence encoding the mutant Ras85D in which glycine is substituted with aspartic acid at 12th position of the amino acid sequence of SEQ ID NO: 1 in the downstream of a UAS sequence,
b') a nucleic acid having a nucleotide sequence encoding shRNA for a p53 gene in the downstream of a UAS sequence,
c') a nucleic acid having a nucleotide sequence encoding a Cyclin E gene in the downstream of a UAS sequence, and
d') a nucleic acid having a nucleotide sequence encoding shRNA for a Med gene in the downstream of a UAS sequence.

Drosophila with the characteristics a) to d) can be produced with reference to production of ptc>dRet^{M955T} described in Non-Patent Literatures 2 to 4. An outline of an example of a production protocol is shown in the following (i) to (iii), but the production method and Drosophila are not limited to the following examples:
(i) modifying a Drosophila genome to produce a transformed Drosophila 1, by using a Drosophila expression vector in which a nucleotide sequence encoding the mutant Ras85D and a nucleotide sequence encoding shRNA for the p53 gene are incorporated under the control of UAS,
(ii) modifying a Drosophila genome to produce a transformed Drosophila 2, by using a Drosophila expression vector in which a nucleotide sequence of the Cyclin E gene and a nucleotide sequence encoding shRNA for the Med gene are incorporated under the control of UAS, and
(iii) mating the transformed Drosophila 1 with the transformed Drosophila 2 to produce a transformed Drosophila 3, and then mating the transformed Drosophila 3 with the gal4 driver line to produce a model Drosophila with the characteristics a) to d).

Drosophila with the characteristics a) to d) can generate tumor cells with abnormal proliferation and enhanced migration capability. These phenomena are considered to result from reproduction of human pancreatic cancer traits. Therefore, Drosophila with the characteristics a) to d) can be used as a model for human pancreatic cancer. It is possible to explore a gene affecting the human pancreatic cancer-like traits that are exhibited in Drosophila with the characteristics a) to d), by investigating the traits and survival rate of F1 obtained by mating the Drosophila having the characteristics a) to d) with a heterozygous mutant Drosophila having a gene of which the function is reduced by introducing a mutation such as deletion into one of alleles of the gene.

### Method for screening anticancer agents

Another aspect of the present disclosure provides a method for screening an anticancer agent comprising the following steps: a step of making a test substance be ingested by Drosophila with the characteristics a) to d); a step of measuring survival rate of Drosophila ingesting the test substance; and a step of selecting the test substance as a candidate for anticancer agent when the survival rate of Drosophila ingesting the test substance is higher than that of Drosophila not ingesting the test substance.

The method for screening an anticancer agent includes a step of making the test substance be ingested by the above human pancreatic cancer-mimicking Drosophila, that is, Drosophila with the characteristics a) to d). This step is typically performed by feeding a food containing the test substance to Drosophila with the characteristics a) to d). The food contains an appropriate amount of the test substance, and can be prepared by a method for preparing a normal food for Drosophila with use of materials for the normal food for Drosophila. The amount of the test substance and the method for mixing with other materials can be determined as appropriate depending on physical properties of the test substance, expected effective concentration and the like. The period for feeding the food is a larval period of Drosophila corresponding to a period in which Drosophila hatches from an egg and pupates. In this step, Drosophila can be reared under the conditions normally used for rearing Drosophila as a laboratory animal, as long as the rearing temperature is adjusted as described below to obtain the desired survival rate and the humidity is maintained to the extent that the food does not dry out.

One of the preferred examples of this step is a step of placing an egg of Drosophila having the characteristics a) to d) on the food containing the test substance and then feeding the food containing the test substance to each of hatched larvae. Another preferred example is a step of placing a larva of Drosophila having the characteristics a) to d) on the food containing the test substance and then feeding the food containing the test substance.

The method for screening an anticancer agent includes a step of measuring survival rate of Drosophila ingesting the test substance, and a step of selecting the test substance as a candidate for anticancer agent when the survival rate of Drosophila ingesting the test substance is higher than that of Drosophila not ingesting the test substance. The survival rate can be calculated by dividing the number of individuals eclosing from pupal cases by the total number of pupae.

Drosophila with the characteristics a) to d) generates tumor cells with abnormal proliferation and enhanced migration capability, and the survival rate decreases. In the case that the gal4-UAS system is used, the probability that the larvae of Drosophila die without eclosing from pupae increases as the rearing temperature is increased within a range of 16 to 29°C. For example, in the case of a Ser-gal4; UAS-Ras^{G12D}, UAS-p53^{shRNA}, UAS-CycE and UAS-Med^{shRNA} 4-hit fly (non-GFP) described in Examples 3 to 5, the survival rate is substantially 0% at 25°C or higher. Therefore, when the survival rate for Drosophila reared with ingesting the test substance is higher than that for Drosophila reared under the same condition without ingesting the test substance, for example, in the case of more than 0% survival rate for Drosophila reared with ingesting the test substance in contrast to 0% survival rate for Drosophila reared under the same condition without ingesting the test substance, the test substance is considered to have the suppressive activity against the expression of human pancreatic cancer-like traits that are exhibited in Drosophila having the characteristics (a) to (d), and therefore can be selected as the candidate substance for anticancer agent for pancreatic cancer.

The survival rate of Drosophila having the characteristics (a) to (d) obtained by utilizing the gal4-UAS system can be controlled by adjusting the rearing temperature to control the expression levels of the above four nucleic acids. For example, in the case of a Ser-gal4; UAS-Ras^{G12D}, UAS-p53^{shRNA}, UAS-CycE, UAS-Med^{shRNA} 4-hit fly (non-GFP) described in Examples 3 to 5, the survival rate is substantially 0% when reared at 25°C or higher, and the survival rate can be raised up to approximately 10 to 20% when reared at 22 to 24°C. The test substance selected by the present screening method performed at the rearing temperature of 22 to 24°C is expected to exhibit a relatively moderate anticancer activity, compared to the test substance selected by the present screening method performed at the rearing temperature of 25°C or higher.

In a preferred embodiment of the present screening method, the method for screening an anticancer agent comprises a step of rearing, on the food containing the test substance, an egg resulting from mating Drosophila into which a gal4 gene is introduced with Drosophila into which the following nucleic acids a') to d') are introduced:
a') the nucleic acid having a nucleotide sequence encoding the mutant Ras85D in which glycine is substituted with aspartic acid at 12th position of the amino acid sequence of SEQ ID NO: 1 in the downstream of a UAS sequence,
b') the nucleic acid having the nucleotide sequence encoding shRNA for a p53 gene in the downstream of a UAS sequence,
c') the nucleic acid having the nucleotide sequence encoding a Cyclin E gene in the downstream of a UAS sequence, and
d') the nucleic acid having the nucleotide sequence encoding shRNA for a Med gene in the downstream of a UAS sequence; and a step of measuring survival rate of Drosophila reared on the food containing the test substance; and a step of selecting the test substance as the candidate substance for the anticancer agent when the survival rate of Drosophila reared on the food containing the test substance is higher than that of Drosophila reared on a food not containing the test substance.

### Pharmaceutical combination

Another embodiment of the present disclosure pertains to a pharmaceutical combination using at least two types of kinase inhibitors selected from the group consisting of a MEK inhibitor, an FRK inhibitor, a WEE inhibitor, an AURK inhibitor and a ROCK inhibitor for treatment of pancreatic cancer.

MEK (MAPK (mitogen-activated protein kinase)/ERK (extracellular signal control kinase) kinase) is a member of protein kinases constituting the MAPK cascade. The MAPK cascade forms a complex signaling network that regulates a wide variety of cellular processes including cell proliferation, growth, differentiation, and apoptosis. Since inhibition of MEK terminates cell proliferation and induces apoptosis, MEK has attracted attentions as a target molecule of anticancer agents, and various MEK inhibitors have been reported.

Examples of the MEK inhibitor used in the present disclosure include Trametinib, Cobimetinib, Binimetinib, Selumetinib, pimasertib, Mirdametinib, Refametinib, PD184352, PD98059, BIX02189, BIX02188, TAK-733, AZD8330, PD318088, Myricetin, BI-847325, GDC-0623, Ro 5126766, PD198306, RO4987655 and HI TOPK 032.

FRK (fyn related kinase; also referred to as RAK) is a nuclear non-receptor tyrosine kinase that belongs to SRC subfamily members. It is known that FRK is highly expressed in breast cancer cells and kidney cancer cells.

Examples of the FRK inhibitor used in the present disclosure include AD80 and RAF709.

AURORA kinase (AURK) is a serine/threonine kinase that regulates cell division. It is known that AURK is involved in separation of centrosomes and formation of bipolar spindles during cell division. There are three isoforms, AURK-A, AURK-B and AURK-C, which are highly homologous in their C-terminal kinase domains. Since AURK is highly expressed in many types of cancer cells, AURK has attracted attentions as a target molecule of anticancer agents, and various AURK inhibitors have been reported.

Examples of the AURK inhibitor used in the present disclosure include Alisertib (AURK-A inhibitor), BI-831266 (AURK-B inhibitor), Barasertib (AURK-B inhibitor), Tozasertib (pan-AURK inhibitor with high AURK-A selectivity), Danusertib (pan-AURK inhibitor), CCT137690 (pan-AURK inhibitor), CCT129202 (pan-AURK inhibitor with high AURK-A selectivity), CCT241736 (AURK-A, B inhibitor), SNS-314 (pan-AURK inhibitor), Hesperadin (AURK-B inhibitor), MK-5108 (AURK-A inhibitor), MLN8054 (AURK-A inhibitor), ZM 447439 (AURK-A, B inhibitor), PF03814735 (AURK-A, B inhibitor), AT9283 (AURK-A, B inhibitor), GSK1070916 (AURK-B, C inhibitor), PHA-680632 (pan-AURK inhibitor with high AURK-A selectivity), Reversine (pan-AURK inhibitor), CYC116 (AURK-A, B inhibitor), ENMD-2076 (AURK-A inhibitor), TAK-901 (AURK-A, B inhibitor), AMG 900 (pan-AURK inhibitor), MK-8745 (AURK-A inhibitor), JNJ-7706621 (AURK-A, B inhibitor), SCH-1473759 (AURK-A, B inhibitor), Ilorasertib (pan-AURK inhibitor with high AURK-B and C selectivity), TCS7010 (AURK-A inhibitor), LY3295668 (AURK-A inhibitor), BI-811283 (AURK-B inhibitor), Chiauranib (AURK-B inhibitor), and NMI-900 (AURK-B, C inhibitor).

WEE kinase is a nuclear tyrosine kinase that negatively regulates cell division through inactivation of the CDK-1/cyclin B complex by phosphorylation. There are three types of WEE kinase, WEE1 (WEE1A), WEE2 (WEE1B) and Myt1 (PKMYT1). WEE1 plays an important role in mitotic G2/M checkpoint, and functions in conjunction with MYT1 to shift cells into G2-M arrest. The WEE1 inhibition in cancer cells inactivates G1 checkpoint to cause chromosome instability, eventually leading to mitotic catastrophe. Thus, WEE kinase, WEE1 in particular, has attracted attentions as a target molecule of anticancer agents.

Examples of the WEE inhibitor used in the present disclosure include Adavosertib (also referred to as MK-1775 or AZD1775), PD0166285 and PD407824.

ROCK (Rho-associated protein kinase) is a serine/threonine kinase that regulates cytoskeleton. There are two isoforms of ROCK, ROCK1 and ROCK2. ROCK is a downstream target of a small GTPase RhoA, and phosphorylates many substrates, and is involved in a wide variety of biological functions such as cell motility, cell polarity, cell adhesion, cell division, apoptosis and transcriptional regulation. Therefore, ROCK proteins have attracted attentions as target molecules of various pharmaceuticals including anticancer agents, and various ROCK inhibitors have been reported.

Examples of the ROCK inhibitor used in the present disclosure include Y-27632 (ROCK1 inhibitor), Fasudil (ROCK2 inhibitor), Azaindole 1 (pan-ROCK inhibitor), AT13148 (pan-ROCK inhibitor), Thiazovivin, Netarsudil, Ripasudil, Chroman 1, GSK429286A, RKI-1447, GSK269962A, Y-39983, KD025, SAR407899, BDP5290, SB-772077B, H-1152, LX7101, SR-3677, Y-33075, CMPD101, and SLx-2119.

The pharmaceutical combination in this aspect refers to a medication containing a combination of at least two types of the kinase inhibitors explained above, that is, a combination of at least two types selected from the group consisting of MEK inhibitors, FRK inhibitors, WEE inhibitors, AURK inhibitors and ROCK inhibitors. The term "combination of at least two types of kinase inhibitors" encompasses a combination of two types of kinase inhibitors, a combination of three types of kinase inhibitors, a combination of four types of kinase inhibitors, and a combination of all five types of kinase inhibitors. The term does not include combinations of at least two inhibitors selected from the same types of kinase inhibitor, for example two or more combinations of kinase inhibitors classified into MEK inhibitors. Therefore, for example, the term "combination of two types of the kinase inhibitors" means a combination of MEK inhibitor and FRK inhibitor, a combination of MEK inhibitor and WEE inhibitor, a combination of MEK inhibitor and AURK inhibitor, a combination of MEK inhibitor and ROCK inhibitor, a combination of FRK inhibitor and WEE inhibitor, a combination of FRK inhibitor and AURK inhibitor, a combination of FRK inhibitor and ROCK inhibitor, a combination of WEE inhibitor and AURK inhibitor, a combination of WEE inhibitor and ROCK inhibitor, and a combination of AURK inhibitor and ROCK inhibitor.

Each inhibitor in the combination is not limited to one compound. For example, a combination of MEK inhibitor and FRK inhibitor is not limited to a combination of one MEK inhibitor and one FRK inhibitor, but includes a combination of one MEK inhibitor and two or more FRK inhibitors, a combination of two or more MEK inhibitors and one FRK inhibitor, and a combination of two or more MEK inhibitors and two or more FRK inhibitors.

The pharmaceutical combination in the present aspect can include any combination of two or more types of kinase inhibitors among the five types of kinase inhibitors. In an embodiment, the combination is a combination of at least two types of kinase inhibitors selected from the group consisting of MEK inhibitor, FRK inhibitor, WEE inhibitor, and AURK inhibitor, the MEK inhibitor being Trametinib, the FRK inhibitor being AD80, the WEE inhibitor being Adavosertib, the AURK inhibitor being Alisertib or BI-831266. The combination may be a combination of a MEK inhibitor and at least one type of kinase inhibitor selected from other four types of kinase inhibitors. In another embodiment, the combination is a combination of Trametinib as MEK inhibitor and at least one type of kinase inhibitor selected from the group consisting of FRK inhibitor, WEE inhibitor, and AURK inhibitor, the FRK inhibitor being AD80, the WEE inhibitor being Adavosertib, the AURK inhibitor being Alisertib or BI-831266.

Two or more types of kinase inhibitors contained in the pharmaceutical combination are administered together or individually, simultaneously or sequentially to a subject in need of treating pancreatic cancer, that is, a subject for whom treatment of pancreatic cancer is desired. The pharmaceutical combination may be in a form of formulation containing two or more types of kinase inhibitors together, and may be in a combination of forms of individual formulations of kinase inhibitors. When the pharmaceutical combination is a combination of individual formulations, the order of administration and the timing of administration for each formulation are not particularly limited, and administration may be performed simultaneously, or at different times, or on different days with a certain time interval.

Another aspect of the present disclosure provides individual kinase inhibitors intended for use in the pharmaceutical combination.

The above pharmaceutical combination can be used for treatment of pancreatic cancer. The term "treatment" encompasses all types of therapeutic interventions medically acceptable for cure or temporary remission of diseases. That is, the treatment of pancreatic cancer encompasses interventions medically acceptable for various purposes including delay or stop of the progression of pancreatic cancer, the retraction or disappearance of lesion, the prevention of recurrence and the like.

The pharmaceutical combination is administered to pancreatic cancer-affected subjects, for example, rodents including mice, rats, hamsters and guinea pigs, primates including humans, chimpanzees, and rhesus monkeys, domestic animals including pigs, cattle, goats, horses and sheep, and companion animals including dogs and cats. Preferred subject is human.

Pancreatic cancer to be treated includes exocrine pancreatic tumors such as invasive pancreatic ductal carcinoma, pancreatic adenocarcinoma, intraductal papillary mucinous tumor, and endocrine pancreatic tumors such as neuroendocrine tumors.

The amount of each kinase inhibitor used in the pharmaceutical combination is such an amount as to treat pancreatic cancer when combined with the other kinase inhibitor(s) for the pharmaceutical combination, and is generally equal to or less than the amount when used alone. The pharmaceutical combination containing each kinase inhibitor in an amount comparable to that used alone can exhibit a stronger effect on pancreatic cancer, and can treat pancreatic cancer in a shorter period of time or in subjects in which use of each kinase inhibitor alone fails to demonstrate an effect. The pharmaceutical combination containing each kinase inhibitor in a smaller amount than that used alone have an advantage that the amounts of the kinase inhibitors can be reduced while maintaining the effect on pancreatic cancer.

The pharmaceutical combination and the kinase inhibitor for the pharmaceutical combination can be used in a form of a pharmaceutical composition containing pharmaceutically acceptable components such as a medicine other than the kinase inhibitor, buffer, antioxidant, preservative, protein, hydrophilic polymer, amino acid, chelating agent, nonionic surfactant, excipient, stabilizer and carrier. The pharmaceutically acceptable components are well known to the person skilled in the art, and can be selected and used as appropriate depending on pharmaceutical formulation from components that are well known to the person skilled in the art and described in Japanese Pharmacopoeia 17th Edition, for example, and other standard documents, within the scope of normal practice capabilities for the person skilled in the art.

The pharmaceutical composition contains effective amounts of the kinase inhibitors. The effective amount described herein refers to such an amount as to treat cancer when combined with the other kinase inhibitor(s) for the pharmaceutical combination, as described above. The effective amount is determined as appropriate depending on type and ratio of each kinase inhibitor to be combined, usage, age of the subject, states of the disease and other conditions.

The dosage form of the pharmaceutical composition can be any form, and preferred examples of the form can include oral dosage form (tablet, capsule, powder, granule, fine granule, pill, suspension, emulsion, liquid, syrup and the like) and parenteral dosage form (injection, drip, enteric agent, transdermal agent and the like). The administration route of the pharmaceutical composition is not particularly limited. Examples of the administration for parenteral dosage form include intravascular administration (preferably intravenous administration), intraperitoneal administration, intestinal administration, subcutaneous administration, and topical administration to a target site. In a preferred embodiment, the pharmaceutical composition is administered by oral administration or intravenous administration.

### Method for treatment of cancer

Another aspect of the present disclosure provides a method for treatment of pancreatic cancer comprising administering to a subject in need thereof effective amounts of at least two types of kinase inhibitors selected from the group consisting of MEK inhibitors, FRK inhibitors, WEE inhibitors, AURK inhibitors and ROCK inhibitors in combination, together or individually, simultaneously or sequentially.

In the description above, the parts not falling within the scope of the claims are disclosed as references.

The present disclosure will be described in more detail with reference to the following Examples.

### EXAMPLES

### Example 1 Production of human pancreatic cancer Drosophila model

Genomic DNA was extracted from Drosophila w⁻(Bloomington Drosophila Stock Center) by a conventional method. PCR was carried out using this genomic DNA as a template as well as primer DNAs designed so as to amplify the Ras85D gene to obtain a DNA fragment containing a nucleotide sequence of the Ras85D gene. Then, primer DNAs for site-directed mutagenesis to substitute the codon encoding glycine with a codon encoding aspartic acid at 12th position of the amino acid sequence of Ras85D were designed and synthesized. PCR was carried out using the DNA fragment as a template as well as the primer DNAs for site-directed mutagenesis to prepare a DNA fragment encoding a Ras85D mutant (Ras^{G12D}). This DNA fragment was inserted into pWALIUM vector (Harvard Medical School) that is a knockdown vector for Drosophila to prepare pWALIUM UAS-Ras^{G12D} vector. This vector was microinjected into Drosophila y¹w^{67c23};P{CaryP}attP2 to produce a UAS-Ras^{G12D} fly with the DNA encoding Ras^{G12D} being inserted in the L region of chromosome 3 by homologous recombination.

In addition, the DNA fragment encoding Ras^{G12D} was inserted into pWALIUM vector, together with a DNA fragment containing a p53 gene knockdown sequence (a sequence connecting sense strand TGCTGAAGCAATAACCACCGA (SEQ ID NO: 2), hairpin loop TAGTTATATTCAAGCATA (SEQ ID NO: 6) and antisense strand TCGGTGGTTATTGCTTCAGCA (SEQ ID NO: 3)) to prepare pWALIUM.UAS-Ras^{G12D},UAS-p53^{shRNA} vector. This vector was microinjected in the same way into Drosophila y¹w^{67c23};P{CaryP}attP2 to produce a UAS-Ras^{G12D}, UAS-p53^{shRNA} fly with the DNA encoding Ras^{G12D} and shRNA for p53 gene being inserted in the L region of chromosome 3.

Further, PCR was carried out using the Drosophila w⁻ genome DNA as a template as well as primer DNAs designed so as to amplify the Cyclin E (CycE) gene to obtain a DNA fragment containing a nucleotide sequence of the CycE gene. This DNA fragment was inserted into pWALIUM vector, together with a DNA fragment containing a Med gene knockdown sequence (a sequence connecting sense strand TTCAGTGCGATGAACATTGCT (SEQ ID NO: 4), hairpin loop TAGTTATATTCAAGCATA (SEQ ID NO: 6) and antisense strand AGCAATGTTCATCGCACTGAA (SEQ ID NO: 5)) to prepare pWALIUM.UAS-CycE,UAS-Med^{shRNA} vector. This vector was microinjected into Drosophila PBac{yellow[+]-attP-9A}VK00027 to produce a UAS-CycE, UAS-Med^{shRNA} fly with the DNA encoding CycE gene and shRNA for Med gene being inserted in the R region of chromosome 3 by homologous recombination.

Next, a UAS-Ras^{G12D}, UAS-p53^{shRNA} fly was mated with a UAS-CycE, UAS-Med^{shRNA} fly at 25°C for 3 days to produce a UAS-Ras^{G12D}, UAS-p53^{shRNA}, UAS-CycE, UAS-Med^{shRNA} fly.

Each of the UAS-Ras^{G12D} fly and the UAS-Ras^{G12D}, UAS-p53^{shRNA}, UAS-CycE, UAS-Med^{shRNA} fly was mated with a ptc-gal4, UAS-GFP fly (ptc>GFP fly, Bloomington Drosophila Stock Center) at 25°C for 3 days to produce a ptc>GFP; UAS-Ras^{G12D} fly (represented as 1-hit fly (ptc>GFP)) and a ptc>GFP; UAS-Ras^{G12D}, UAS-p53^{shRNA}, UAS-CycE, UAS-Med^{shRNA} fly (represented as 4-hit fly (ptc>GFP)). In these flies, a transgene was induced to express in epithelial cells localized to a wing disc under control of the ptc promoter.

Fig. 1 shows fluorescent microscope images of the wing discs of 3rd instar larvae of a 1-hit fly (ptc>GFP), a 4-hit fly (ptc>GFP) and a control ptc>GFP fly. In the control, GFP expression was observed in monolayer epithelial cells with a width of approximately 10 cells (images in second leftmost and the middle). The 1-hit fly (ptc>GFP) showed GFP-expressing cells with a wider width (second rightmost image). In the 4-hit fly (ptc>GFP) (rightmost image), this tendency is more remarkable , and the appearance of tumor cells (indicated by arrowheads in the figure) with enhanced migration capability that had departed from original region was observed.

After the mating to produce the 1-hit fly (ptc>GFP) and the 4-hit fly (ptc>GFP), the parent flies were left to lay eggs on a food for two days to obtain 20 to 50 eggs per vial. The eggs were reared at 16°C for 25 days, and then the survival rate was calculated by dividing the number of eclosed pupae by the total number of pupae. While the survival rate of the control ptc>GFP flies was 100%, that of the 1-hit flies (ptc>GFP) was 55%, and that of the 4-hit flies (ptc>GFP) was 0%, i.e. lethal.

These results reveal that the Ras^{G12D} expression enhances proliferation of the wing disc epithelial cells, and that the proliferation of the wing disc epithelial cells was enhanced further by inductions of p53 knockdown, enhancement of CycE expression and Med gene knockdown in addition to the Ras^{G12D} expression as well as that the migration capability is also enhanced, thereby demonstrating that a 4-hit fly can be a Drosophila model for human pancreatic cancer.

### Example 2 Search of kinase genes influencing 4-hit fly survival rate

In accordance with methods described in Sonoshita et al. (Curr. Top. Dev. Biol., 2017, 121, 287-309), the mating was carried out as shown in Fig. 2, and then the kinase genes influencing the survival rate of 4-hit flies were searched. Specifically, the UAS-Ras^{G12D}, UAS-p53^{shRNA}, UAS-CycE, UAS-Med^{shRNA} fly produced in Example 1 was mated with an SM5_{tubgal80}-TM6B balancer fly (Dr. Ross Cagan, Icahn School of Medicine at Mount Sinai, NY, USA) to produce a UAS-Ras^{G12D}, UAS-p53^{shRNA}, UAS-CycE, and UAS-Med^{shRNA}/SM5_{tubgal80}-TM6B fly. Next, this fly was mated with a Ser>GFP fly (Bloomington Drosophila Stock Center) to produce a Ser>GFP; UAS-Ras^{G12D}, UAS-p53^{shRNA}, UAS-CycE, UAS-Med^{shRNA}/SM5_{tubgal80}-TM6B fly.

Two hundred and twenty available heterozygous mutant lines of kinases in the whole fly kinome were obtained from the Bloomington Drosophila Stock Center (USA), and each of these was mated with a Ser>GFP; UAS-Ras^{G12D}, UAS-p53^{shRNA}, UAS-CycE, UAS-Med^{shRNA}/SM5_{tubgal80}-TM6B fly at 27°C for 3 days to obtain eggs of a 4-hit fly with a heterozygous kinase mutation. As a control, a w⁻ fly was mated with a Ser>GFP; UAS-Ras^{G12D}, UAS-p53^{shRNA}, UAS-CycE, UAS-Med^{shRNA}/SM5_{tubgal80}-TM6B fly to obtain eggs of a 4-hit fly without a kinase mutation. These flies were reared at 27°C for 13 days, and then survival rate was calculated by dividing the number of eclosed pupae by the total number of pupae.

A comprehensive search of fly kinase genes revealed that each of heterozygous mutations of the RAS pathway effector MEK, SRC family kinase FRK, mitotic regulators WEE and AURORA, and cytoskeleton regulator ROCK suppressed the lethality of 4-hit flies (Fig. 3). Fig. 3 shows the survival rate of these heterozygous mutant flies. This suggests that the use of MEK inhibitor, FRK inhibitor, WEE inhibitor, AURK inhibitor and ROCK inhibitor can be effective to increase the survival rate of 4-hit flies.

### Example 3 Evaluation of efficacy of kinase inhibitor on 4-hit fly survival rate

Trametinib (MEK inhibitor), Adavosertib (referred also to as MK-1775, WEE1 inhibitor), AD80 (FRK inhibitor), Y-27632 (ROCK inhibitor), Alisertib (AURKA inhibitor, the above all purchased from MedChem Express), BI-831266 (AURKB inhibitor, obtained from Boehringer Ingelheim) was each dissolved in DMSO (SIGMA) and stored at -20°C.

Agar (Wako), brewers' yeast (MP Bio), yeast extract (Sigma Aldrich), bacto casitone (BD), sucrose (Wako), glucose (Wako), MgCl₂ (Wako) CaCl₂ (Wako), Propionic acid (Wako), and Mold inhibitor (10% Methyl-4-hydroxy Benzoate in 95% Ethanol; Wako) were dissolved in ultrapure water to prepare a standard food. While kept at 50°C, the kinase inhibitor (Trametinib alone or a combination of Trametinib and another kinase inhibitor) dissolved in DMSO was added for mixing. The resulting solution was cooled to obtain a drug food. The final concentrations of the compounds in the food were 1 µM for Trametinib, 100 µM for Adavosertib, 50 µM for AD80, 10 µM for Y-27632, and 100 µM for BI-831266.

The UAS-Ras^{G12D}, UAS-p53^{shRNA}, UAS-CycE, UAS-Med^{shRNA} fly produced in Example 1 was mated with a Ser-gal4 fly (Bloomington Drosophila Stock Center). Then, the parent flies were left to lay eggs on the standard food or the drug food for two days to obtain 20 to 50 eggs of Ser-gal4; UAS-Ras^{G12D}, UAS-p53^{shRNA}, UAS-CycE, UAS-Med^{shRNA} 4-hit flies (non-GFP) per vial. The eggs were reared at 25°C for 13 days, and survival rate of 4-hit flies (non-GFP) reared on each food was calculated by dividing the number of eclosed pupae by the total number of pupae.

Trametinib used alone increased survival rate of 4-hit flies (non-GFP) by 20%. None of other kinase inhibitors used alone changed survival rate of 4-hit flies (non-GFP). However, when combined with Trametinib, other kinase inhibitors further improved the survival rate compared to Trametinib used alone, suggesting a synergistic effect resulting from the combination of Trametinib and another kinase inhibitor (Fig. 4).

### Example 4 Evaluation of efficacy of kinase inhibitor with use of human pancreatic cancer cells

Human pancreatic cancer cell line (MIA PaCa-2) was purchased from National Institute of RIKEN BioResource Research Center, and cultured in Dulbecco's Modified Eagle's Medium (Nacalai tesque) supplemented with 10% fetal bovine serum (Gibco) and 1% penicillin-streptomycin (Nacalai tesque) at 37°C in the presence of 5% CO₂.

Trametinib (DMSO only, 1 µM, 300 µM), Adavosertib (300 µM, 1 mM), AD80 (30 µM, 3 mM), Alisertib (30 µM, 1 mM) and BI-831266 (3 µM, 3 mM) was each prepared and diluted to 100-fold in the medium to produce a drug solution.

MIA PaCa-2 cells were seeded into a 96-well plate at 1000 cells/well (in 100 µL medium). After culturing for one day, 10 µL of the drug solution of Trametinib alone or Trametinib combined with another kinase inhibitor was added to the cells in each well on the 96-well plate (0.2% final concentration of DMSO). Each combination was tested in five wells. After the addition of the drug solution, the cells were cultured at 37°C for 72 hours in the presence of 5% CO₂, and then the cell viability was measured using MTS assay (Cell Titer 96 (registered trademark), Promega). The viability is expressed as a ratio to that of a control (solvent only).

Trametinib used alone reduced the cell viability in a dose-dependent manner. Each of the combination Trametinib and any one of Adavosertib, AD80, Alisertib and BI-831266 greatly reduced the cell viability compared to that of Trametinib used alone, suggesting a synergistic effect resulting from the combination between Trametinib and another kinase inhibitor (Fig. 5).

### Example 5 Evaluation of temperature dependency of lethality of 4-hit flies

4-hit flies (non-GFP) produced in Example 3 were reared at different temperatures of 22, 24, 25, 27 and 29°C. The survival rate decreased with increase in the temperature, and those reared at temperatures of 25°C or higher became dead (Fig. 6).

### Example 6 Evaluation of efficacy of kinase inhibitor with use of cancer-bearing mice

BALB/c-nu/nu immunodeficient nude mice (6 weeks old, specific pathogen-free rearing) were anesthetized, and 5 × 10⁶ MIA PaCa-2 cells were subcutaneously implanted to the mice. The mice were kept while measuring tumor volume (long diameter × short diameter × short diameter/2) until the volume reached 100 mm³, and then the mice were divided into four groups (n=8). Solvent (5% DMSO), Trametinib (1 mg/kg body weight/day), BI-831266 (10 mg/kg body weight/day), or Trametinib (1 mg/kg body weight/day) and BI-831266 (10 mg/kg body weight/day) was orally administered to mice in each group for 5 days per week, and tumor volume was measured over time.

In the group (T+B group) to which the combination of Trametinib and BI-831266 was administered, the increase in mean tumor volume was significantly reduced, compared to those in the group (T group) to which Trametinib alone was administered and the group (B group) to which BI-831266 alone was administered (Fig. 7A). The majority of mice exhibited partial remission (reduction in tumor volume by 30% or more) or complete remission (tumor disappearance) in the combined administration groups, whereas none of mice exhibited partial or complete remission in the group to which solvent was administered and the group to which Trametinib alone was administered. Only one mouse exhibited complete remission in the BI-831266 administered group (Fig. 7B). These results demonstrated that the combination of Trametinib and BI-831266 exhibited a synergistic suppressive effect on cell proliferation of human pancreatic cancer cells implanted into mice, and that there was little individual variability in the effect.

In the Examples above, the parts which do not fall within the scope of the claims are disclosed as references.

### [Sequence table free text]

SEQ ID NO: 1 Amino acid sequence of Ras85D
SEQ ID NO: 2 Nucleotide sequence of DNA that encodes sense strand in shRNA targeting p53 gene
SEQ ID NO: 3 Nucleotide sequence of DNA that encodes antisense strand in shRNA targeting p53 gene
SEQ ID NO: 4 Nucleotide sequence of DNA that encodes sense strand in shRNA targeting Med gene
SEQ ID NO: 5 Nucleotide sequence of DNA that encodes antisense strand in shRNA targeting Med gene
SEQ ID NO: 6 Nucleotide sequence of DNA encoding shRNA hairpin loop

## Claims

1. A pharmaceutical combination of Trametinib and at least one kinase inhibitor selected from the group consisting of AD80, BI-831266 and Y-27632 for use in the treatment of pancreatic cancer.

2. A kinase inhibitor for use in combination with Trametinib for use in the treatment of pancreatic cancer, the kinase inhibitor being selected from the group consisting of AD80, BI-831266 and Y-27632.

3. Trametinib for use in combination with at least one type of kinase inhibitor selected from the group consisting of AD80, BI-831266 and Y-27632, for use in the treatment of pancreatic cancer.

## Patentansprüche

1. Pharmazeutische Kombination aus Trametinib und mindestens einem Kinase-Inhibitor, ausgewählt aus der Gruppe bestehend aus AD80, BI-831266 und Y-27632, zur Verwendung bei der Behandlung von Pankreaskrebs.

2. Kinase-Inhibitor zur Verwendung in Kombination mit Trametinib zur Verwendung bei der Behandlung von Pankreaskrebs, wobei der Kinase-Inhibitor aus der Gruppe bestehend aus AD80, BI-831266 und Y-27632 ausgewählt ist.

3. Trametinib zur Verwendung in Kombination mit mindestens einem Typ von Kinase-Inhibitor, ausgewählt aus der Gruppe bestehend aus AD80, BI-831266 und Y-27632, zur Verwendung bei der Behandlung von Pankreaskrebs.

## Revendications

1. Une combinaison pharmaceutique de tramétinib et d'au moins un inhibiteur de kinase choisi parmi le groupe constitué de l'AD80, le BI-831266 et le Y-27632, pour utilisation dans le traitement du cancer du pancréas.

2. Un inhibiteur de kinase pour utilisation en combinaison avec le tramétinib pour utilisation dans le traitement du cancer du pancréas, ledit inhibiteur de kinase étant choisi parmi le groupe constitué de l'AD80, le BI-831266 et le Y-27632.

3. Le tramétinib, pour utilisation en combinaison avec au moins un type d'inhibiteur de kinase choisi parmi le groupe constitué de l'AD80, le BI-831266 et le Y-27632, pour utilisation dans le traitement du cancer du pancréas.
